# FASCICULE DE BREVET EUROPEEN

(11) **EP 2 317 946 B1**
(45) Date de publication et mention de la délivrance du brevet: **21.03.2012**
(21) Numéro de dépôt: 09806495.9
(22) Date de dépôt: 17.07.2009
(51) Int. Cl.: A61B 17/70

(54) **ENSEMBLE EXTRA-DISCAL DE STABILISATION INTERVERTEBRALE POUR ARTHRODESE**
EXTRADISKALES BANDSCHEIBENSTABILISIERUNGSELEMENT FÜR ARTHRODESE
EXTRA-DISCAL ASSEMBLY FOR INTERVERTEBRAL STABILISATION FOR ARTHRODESIS

(30) Priorité: 14.08.2008 FR 0855574
(43) Date de publication de la demande: 11.05.2011
(73) Titulaire: Graf, Henry, 69006 Lyon (FR)
(72) Inventeur: Graf, Henry, 69006 Lyon (FR)
(74) Mandataire: Myon, Gérard Jean-Pierre
(86) Numéro de dépôt international: PCT/FR2009/000880
(87) Numéro de publication internationale: WO 2010/018316

(56) Documents cités:
- EP-A- 0 667 127
- FR-A- 2 701 650
- FR-A- 2 709 247
- FR-A- 2 751 864

## Description

La présente invention concerne un ensemble extra-discal de stabilisation intervertébrale, pour arthrodèse.

L'invention se situe dans le domaine de l'arthrodèse, à savoir de la fusion osseuse entre au moins deux vertèbres adjacentes. On rappellera que l'arthrodèse vise à autoriser uniquement des micromouvements entre les vertèbres, ainsi qu'un amortissement des vibrations. Ces micromouvements permettent entre autres au patient, redevenu bipède après l'opération, d'adapter son équilibre au mieux, avant la prise de la greffe osseuse. Ces micromouvements permettent également à l'ensemble conforme à l'invention, de ne pas s'opposer après la fusion osseuse à des adaptations plastiques, qui sont l'une des caractéristiques fondamentales de l'évolution de la colonne vertébrale au cours de la vie du patient.

De manière typique, un ensemble extra-discal pour arthrodèse au sens de l'invention autorise une amplitude de mouvement entre deux vertèbres, vues de côté, qui est égale au plus à environ 10% de l'amplitude physiologique naturelle. En d'autres termes, s'il existe une amplitude naturelle maximale de 10° en rotation entre deux vertèbres adjacentes données, l'ensemble de stabilisation conforme à l'invention est susceptible d'autoriser, au plus, un débattement de 1 ° entre ces deux vertèbres.

On notera qu'il est fondamental d'opérer une distinction entre, d'une part, une prothèse qui est conçue pour recréer le mouvement intervertébral et, d'autre part, l'ensemble selon l'invention dont le seul but est d'obtenir une fusion osseuse entre deux vertèbres. A cet égard, on rappellera qu'il existe des différences structurelles et fonctionnelles substantielles entre une prothèse et un dispositif d'ostéosynthèse, de sorte qu'une prothèse ne peut assurer la fusion osseuse et que, de manière similaire, un ensemble pour arthrodèse ne peut assurer une fonction de prothèse.

L'ensemble de stabilisation conforme à l'invention est destiné à relier au moins deux vertèbres adjacentes, tout en étant généralement placé sur un unique côté de la colonne vertébrale, à savoir à droite ou à gauche. L'implantation de cet élément de stabilisation est de type extra-discal, à savoir que celui-ci peut être situé en arrière, mais aussi en avant, de l'espace intervertébral.

On connaît déjà un ensemble de stabilisation, tel que celui proposé par la Société MEDTRONIC sous la référence commerciale AGILE. Cet ensemble comprend deux organes rigides, propres à coopérer avec deux vis pédiculaires implantées dans deux vertèbres adjacentes. Il est en outre prévu un tampon d'amortissement, qui est rapporté contre les plaques d'extrémité des organes rigides, notamment par vulcanisation à chaud. Dans ces conditions ce tampon peut, non seulement être comprimé, mais également être étiré tout en restant solidaire de ces deux organes, grâce à la présence de cette liaison.

Cet ensemble de stabilisation AGILE comprend en outre des moyens de centrage, destinés à éviter que les deux organes rigides ne se désaxent, en particulier lors des mouvements de flexion. A cet effet, on fait appel à un câble solidaire d'un des deux organes rigides, qui traverse le tampon amortissant et s'étend dans le volume intérieur de l'organe rigide creux en regard.

Cette solution connue présente cependant certains inconvénients. En particulier, l'ensemble de stabilisation AGILE a tendance à travailler de manière beaucoup trop importante en flexion, ce qui réduit son efficacité et sa durée de vie. On notera également que cet objet, qui est initialement conçu comme une prothèse d'accompagnement du mouvement vertébral, a été transformé en dispositif d'ostéosynthèse, du fait des contraintes administratives sur le territoire américain. Une telle transformation a été mise en oeuvre sans modification structurelle de cet objet, en vue de brider le mouvement, ce qui met en évidence son impossibilité à obtenir une arthrodèse, puisque cet objet autorise une quantité de mouvements beaucoup trop importante.

On connaît par ailleurs des ensembles de stabilisation commercialisés sous les références DYNESIS et NFLEX. Ces systèmes de stabilisation dynamique postérieurs utilisent les propriétés mécaniques élastiques de tampons élastomères, afin de limiter la mobilité des vis pédiculaires lors du mouvement intervertébral. Dans ces conditions, le déplacement de chaque vis est la résultante de la déformation par compression d'un de ces tampons, pour la flexion si le tampon est à l'extérieur de l'espace entre les vis, ou bien pour l'extension si le tampon est situé entre ces vis.

L'inconvénient principal de ces dispositifs connus réside dans l'absence d'articulation entre la vis et le tampon. De ce fait, lorsque la vis est entraînée en rotation dans le cadre du mouvement de flexion-extension, elle impose au tampon un travail en flexion. Dans ces conditions, ce tampon ainsi sollicité impose à son tour une rotation â la vis, qui ne se situe pas dans le cadre du mouvement intervertébral physiologique naturel.

Les documents FR-A-2 701 650, FR-A-2 751 864 et EP-A-0 667 127 divulguent chacun un dispositif de stabilisation vertébrale pour arthrodèse composé de deux vis vertébrales reliées par un élément de liaison équipé de tampons élastiques permettant de limiter les mouvements des vis vertébrales. Dans ces dispositifs, les vis vertébrales sont pourvues de têtes sphériques articulées avec l'élément de liaison. Cependant, en service, ces dispositifs manquent de stabilité.

De plus, si l'on souhaite se rapprocher du mouvement physiologique naturel au moyen de ces dispositifs, il est nécessaire d'augmenter de manière très notable la raideur des tampons. Dans ces conditions, ces dispositifs se rapprochent des systèmes rigides, ce qui induit des conséquences sur la sollicitation des vis implantées, ainsi que des insuffisances d'absorption des chocs et des vibrations. Les tampons se comportent alors davantage comme des butées, plutôt que des amortisseurs, seule la flexibilité du système permettant d'obtenir un mouvement résiduel.

Ceci étant précisé, l'invention vise à remédier à ces différents inconvénients. A cet effet, elle a pour objet un ensemble extra-discal de stabilisation intervertébrale pour arthrodèse, comprenant
- au moins deux tiges, dites tiges vertébrales, propres à coopérer avec au moins deux vertèbres différentes ;
- au moins deux couples de mâchoires, chaque couple de mâchoires étant placé au voisinage d'une tige correspondante ;
- au moins deux glissières définissant un axe longitudinal dudit ensemble,
- des moyens de rappel d'au moins une mâchoire parmi chaque couple de mâchoires en direction d'une tige vertébrale correspondante,
- chaque tige vertébrale étant propre à déplacer en service au moins une mâchoire, le long des glissières, à l'encontre des moyens de rappel, chaque tige vertébrale étant en outre articulée par rapport à chaque mâchoire.

Conformément à l'invention, les deux glissières ne sont pas alignées et au moins une mâchoire parmi chaque couple de mâchoires est propre à coulisser le long d'au moins deux glissières, selon leur axe longitudinal.

Selon d'autres caractéristiques :
- ladite articulation entre la tige et chaque mâchoire s'exerce selon un unique point de contact entre cette tige et les parois en regard de chaque mâchoire.
- au moins une mâchoire comprend une branche de liaison recourbée selon un rayon de courbure supérieur au rayon de courbure de la tige.
- la branche recourbée est solidaire de deux plots, montés sur deux glissières, éventuellement de manière coulissante.
- les moyens de rappel comprennent deux ressorts, dont chacun est monté sur une glissière correspondante, ces deux ressorts étant propre à rappeler une branche recourbée contre la tige vertébrale.
- au moins une mâchoire comprend un téton d'articulation contre une tige vertébrale correspondante.
- les moyens de rappel comprennent un corps souple massif et le téton appartient à un flanc rigide, solidaire de ce corps souple.
- au moins un couple de mâchoires comprend une première mâchoire montée coulissante le long des glissières, à l'encontre des moyens de rappel, ainsi qu'une seconde mâchoire montée fixe sur les glissières.
- au moins un couple de mâchoires comprend une première et une seconde mâchoires montées coulissantes le long des glissières, alors que les moyens de rappel comprennent un premier et un second moyen de rappel, chaque mâchoire mobile étant propre à coulisser à l'encontre d'un moyen de rappel correspondant.
- le premier et le second moyens de rappel sont confondus en un moyen de rappel unique, comprenant un organe de rappel central, bordé par deux flancs dont chacun définit une première mâchoire respective, il est prévu deux paires de glissières, chaque paire définissant une seconde mâchoire respective, et chaque paire de glissières est propre à coulisser par rapport au flanc adjacent à la mâchoire définie par ces glissières, cette paire de glissières étant en revanche solidaire en translation du flanc opposé, au moins dans le sens d'un rapprochement des deux flancs l'un vers l'autre l'organe de rappel central est un ressort hélicoïdal. - chaque tige appartient à une vis vertébrale correspondante, en particulier à une vis pédiculaire.
- chaque tige est solidaire d'un élément de liaison, propre à relier deux vis vertébrales destinées à être implantées dans un même étage vertébral.
- cet ensemble comprend des moyens de limitation de la course entre deux tiges adjacentes.
- les moyens de limitation de la course sont des moyens de limitation de la compression d'un corps déformable, appartenant aux moyens de rappel.
- les moyens de limitation de la compression comprennent une chambre rigide, s'étendant à distance des parois du corps déformable, en position de repos de ce dernier.
- les moyens de limitation de la compression comprennent au moins une bague entourant une partie du corps déformable, de manière à limiter sa zone de déformation.
- L'ensemble comprend des moyens permettant d'éviter le glissement des glissières et des mâchoires le long des tiges, le long de l'axe principal de ces dernières, dans au moins un sens.
- deux glissières sont propres à guider les tiges, notamment par coopération d'une zone de ces tiges dont la section transversale correspond sensiblement à la distance séparant ces glissières.

L'invention va être décrite ci-après, en référence aux dessins annexés, donnés uniquement à titre d'exemple non limitatif, dans lesquels :
- la figure 1 est une vue en perspective, illustrant un ensemble de stabilisation conforme à un premier mode de réalisation de l'invention ;
- les figures 2 et 3 sont des vues de côté, illustrant la mise en oeuvre de l'ensemble de la figure 1 ;
- les figures respectivement 4 et 7 sont des vues en perspective, analogues à la figure 1, illustrant deux variantes de réalisation de l'invention ;
- les figures 5 et 6, ainsi que 8 et 9, sont des vues de côté analogues aux figures 2 et 3, illustrant la mise en oeuvre respective des ensembles des figures 4 et 7 ;
- la figure 10 est une courbe, illustrant la variation de l'inclinaison intervertébrale en fonction de la force appliquée par le patient, dans différents modes de réalisation de l'invention ;
- la figure 11 est une courbe, analogue à la figure 10, illustrant cette variation selon une autre mise en oeuvre de l'invention ;
- les figures 12 et 13 dont des vues, respectivement en perspective et de côté, illustrant un mode de réalisation supplémentaire de l'ensemble de stabilisation conforme à l'invention ;
- la figure 14 est une vue en perspective, illustrant un ensemble de stabilisation conforme à une variante supplémentaire de réalisation de l'invention ;
- la figure 15 est une vue de côté, illustrant une variante de réalisation de l'ensemble de la figure 14 ;
- les figures 16, 17 et 18 sont des vues en perspective, illustrant un ensemble extra-discal conforme à une variante supplémentaire de l'invention ;
- la figure 19 est une vue en perspective, illustrant la mise en oeuvre de l'ensemble des figures 16 à 18 ;
- la figure 20 est une vue en perspective éclatée, illustrant différents éléments constitutifs d'une variante supplémentaire de réalisation de l'invention ;
- les figures 21 et 22 sont des vues en coupe longitudinale, illustrant l'ensemble de la figure 20, dans deux positions d'utilisation ;
- les figures 23, 24 et 25 sont des vues respectivement en perspective éclatée et en coupe longitudinale, analogues aux figures 20 à 22, illustrant une variante supplémentaire de réalisation de l'invention, et
- la figure 26 est un graphe, analogue à la figure 10, illustrant la variation de l'inclinaison intervertébrale en fonction de la force appliquée, pour le mode de réalisation des figures 20 à 22.

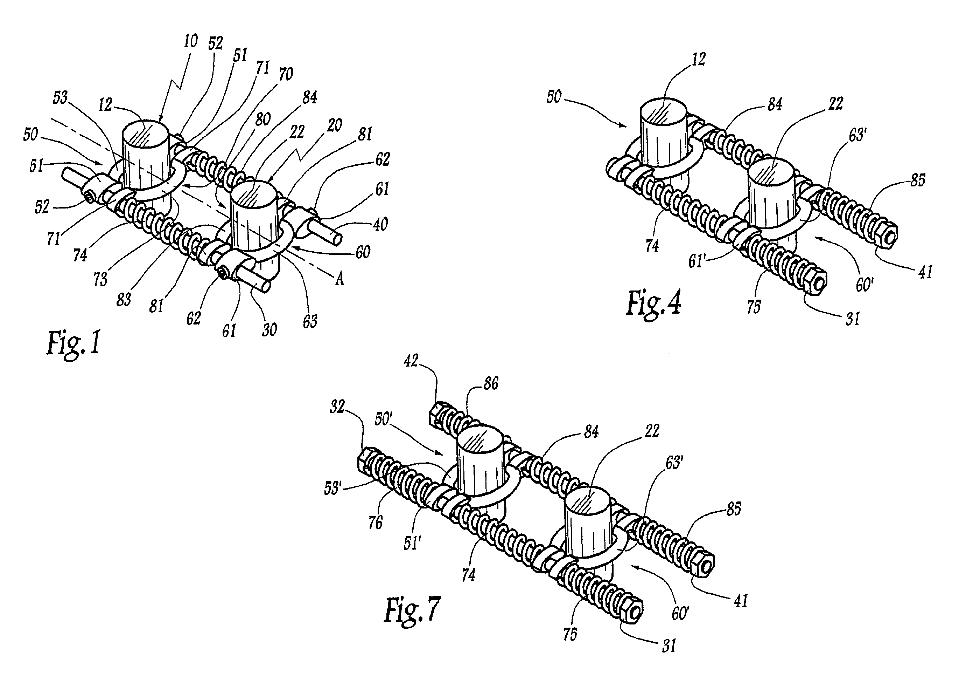

L'ensemble extra-discal conforme à l'invention, illustré sur la figure 1, comprend tout d'abord deux vis vertébrales, qui sont illustrées de manière partielle. Ces deux vis, respectivement affectées des références 10 et 20, comprennent un fût cylindrique 12, 22, plus particulièrement visible sur la figure 1. Ces deux vis sont par exemple des vis pédiculaires comprenant, de manière classique, une zone filetée destinée à pénétrer dans le corps vertébral. Cependant, on peut prévoir d'utiliser, non pas une vis de type pédiculaire, mais tout autre type de vis vertébrale.

Ainsi, cette vis peut être implantée dans le corps vertébral, soit latéralement, soit antérieurement, soit dans le corps vertébral à travers le pédicule. De façon générale, on peut prévoir toute insertion qui assure à la vis une solidarisation stable avec la vertèbre. Elle est alors implantée dans la vertèbre par un filetage et fait dépasser, à l'extérieur de la vertèbre, un téton qui coopère avec une mâchoire, telle que cela sera décrit ci-après. Ce téton peut être également supporté par un organe mécanique différent d'un filetage, telle que par exemple une agrafe ou des crochets placés sur le corps vertébral et/ou les lames osseuses intervertébrales.

L'ensemble de stabilisation conforme à l'invention comprend en outre deux glissières 30 et 40, s'étendant sensiblement selon l'axe A, reliant les deux vis 10 et 20, une fois ces dernières implantées dans les vertèbres. Chaque glissière est constituée d'une tige correspondante 30, 40 qui peut être rigide, ou bien présenter une légère possibilité de déformation, dans le sens de la flexion. En tout état de cause, dans ce dernier cas, cette possibilité de déformation est contrôlée. Lorsqu'elle est vue de côté, qu'elle soit rigide ou non, chaque tige peut être droite, ou bien présenter une légère courbure, afin de s'adapter le cas échéant à la courbure intervertébrale.

Chaque vis 10 ou 20 est associée à deux mâchoires, dont l'une est fixe et l'autre est montée coulissante par rapport aux deux tiges 30 et 40. On note 50 et 60 les deux mâchoires fixes, ainsi que 70 et 80 les deux mâchoires mobiles.

Les quatre mâchoires présentent la même structure, à savoir que chacune d'elle comprend deux plots 51, 61, 71, 81 d'accrochage sur une tige correspondante. Les plots 51 et 61 des mâchoires fixes sont pourvus d'une vis 52, 62, permettant la solidarisation de ce plot sur les deux tiges 30 et 40.

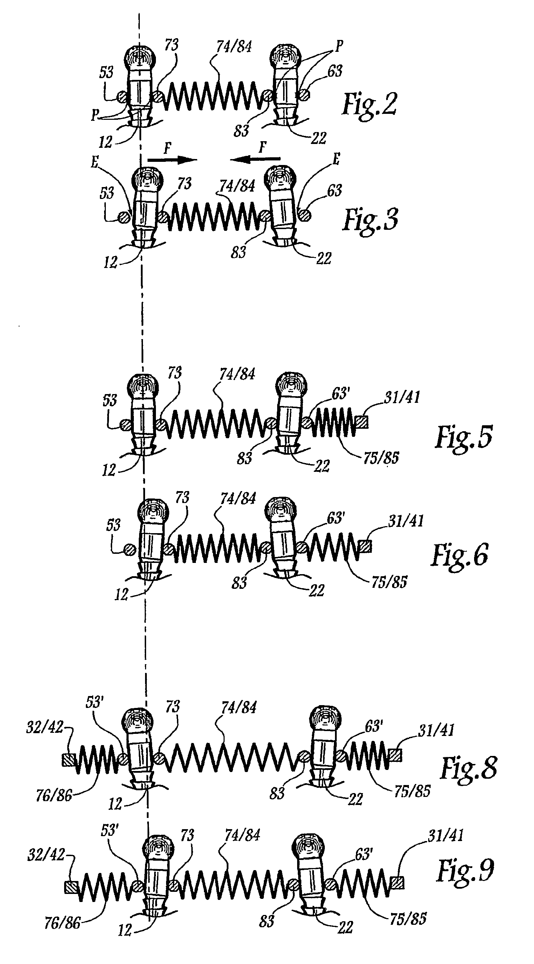

De plus, chaque couple de plots en regard est relié, par l'intermédiaire d'une branche de liaison respective 53, 63, 73 et 83. Chaque branche de liaison est recourbée, en vue de dessus, à savoir selon l'axe de chaque vis, de manière à former un contact articulé avec le fût d'une vis correspondante. En vue de dessus, chaque vis présente un rayon de courbure correspondant à son diamètre alors que chaque branche recourbée représente un rayon de courbure, supérieur au diamètre de cette dernière, ce qui permet cette articulation. De plus, comme le montre la figure 2, chaque branche est circulaire en section transversale, ce qui assure également l'articulation de la mâchoire par rapport au fût cylindrique de la vis.

Chaque branche peut être rigide, ou bien être susceptible de se déformer, au moins par endroits, sous l'effet de contraintes dont l'intensité est bien supérieure à la gravité. En revanche, toutes les branches présentent une forme propre, à savoir que leur forme est invariante sous l'effet de la gravité, ainsi que d'autres contraintes d'intensité analogue.

On notera en outre que les deux mâchoires mobiles 70 et 80 sont adjacentes, à savoir qu'elles sont disposées sur les côtés en regard des deux fûts. En revanche, les deux mâchoires fixes sont éloignées l'une de l'autre, à savoir qu'elles sont présentes sur les faces opposées des deux fûts 12 et 22.

Deux ressorts 74 et 84 sont interposés entre les deux mâchoires mobiles en regard 70 et 80. Ces ressorts tendent à repousser chaque mâchoire mobile 70 ou 80, en direction de la mâchoire fixe 50 ou 60 qui lui est associée.

On notera que, plus ces ressorts sont raides et/ou précontraints, plus ils tendent à s'opposer au mouvement des mâchoires mobiles. La distinction entre ces notions respectives de raideur et de précontrainte sera expliquée plus en détail dans ce qui suit. Dans le cadre de la présente invention, à savoir l'arthrodèse, les ressorts utilisés sont donc d'une raideur et/ou d'une précontrainte importante(s), afin d'autoriser uniquement des micromouvements et un amortissement des vibrations, comme cela a été défini ci-dessus.

Chaque fût 12 ou 22 est circulaire, en section transversale. De plus, chaque branche est également circulaire, également selon une section transversale. Enfin, comme on l'a vu ci-dessus, les branches présentent un profil recourbé, à savoir que les deux branches en regard définissent à peu près un ovale, dont le rayon de courbure est supérieur au rayon du fût circulaire.

Dans ces conditions, chaque fût 12 ou 22 définit, avec chaque branche 53, 73, 63 ou 83, une articulation sensiblement ponctuelle, illustrée par les points P sur la figure 2. En d'autres termes, il existe trois degrés de liberté, en rotation, entre chaque branche et le fût avec lequel elle coopère. Sur cette figure 2, les ressorts 74 et 84 sont illustrés de façon schématique. A titre de variante non représentée, cette articulation peut être réalisée par l'intermédiaire d'un contact non ponctuel, de type méplat sur méplat. Ces deux méplats, dont l'un appartient au fût et dont l'autre appartient à la branche, définissent une zone de contact relativement faible, qui autorise une possibilité de sub-luxation, assimilée à l'articulation au sens de la présente invention.

Le fonctionnement de l'ensemble de stabilisation, décrit ci-dessus, va maintenant être explicité. On suppose que les deux vis 10 et 20 sont des vis pédiculaires et que la partie supérieure de ces vis, sur la figure 1, est dirigée vers l'arrière du patient lorsque celui-ci se tient debout.

Il s'agit tout d'abord de placer les mâchoires autour des vis 10 et 20. On suppose dans le présent exemple que l'ensemble de stabilisation conforme à l'invention est destiné à former un hauban. Dans ces conditions, en l'absence de contrainte, les extrémités libres des deux vis sont séparées selon une distance, qui est supérieure à celle séparant ces vis une fois associées aux mâchoires.

En d'autres termes, en vue du montage, il s'agit tout d'abord de rapprocher mutuellement ces deux vis, par exemple au moyen d'un outil non représenté. On rapproche ensuite axialement les deux mâchoires, ainsi que les deux tiges, par rapport aux vis, de manière à introduire les deux fûts 12 et 22 au travers des deux oeillets, définis par les différentes mâchoires. On relâche ensuite l'action extérieure exercée par l'outil, de sorte que les fûts 12 et 22 viennent se plaquer contre les mâchoires fixes 50 et 60.

Si le patient souhaitait exercer une flexion intervertébrale, à savoir se pencher vers l'avant, les deux vis 10 et 20 auraient tendance à s'écarter l'une de l'autre, selon l'axe A. Ceci est cependant rendu impossible, car les deux fûts 12 et 22 viennent en butée contre les mâchoires fixes 50 et 60. L'ensemble de stabilisation conforme à l'invention empêche donc sensiblement tout mouvement de flexion intervertébrale.

En revanche, lors d'une extension intervertébrale, les vis 10 et 20 ont tendance à se rapprocher l'une vers l'autre, selon l'axe A. Contrairement à la flexion, ce mouvement relatif des vis est possible, dans la mesure où ces dernières repoussent alors les mâchoires mobiles 70 et 80 l'une en direction de l'autre, à l'encontre des ressorts 74 et 84. Ces mâchoires mobiles ont par conséquent tendance à coulisser le long des tiges 30 et 40.

Ceci est plus particulièrement illustré sur la figure 3, où on retrouve les quatre branches, les deux fûts, ainsi que les ressorts 74 et 84. On note que, lors de cette extension intervertébrale, les fûts 12 et 22 se déplacent, non seulement en rotation, mais également en translation selon les flèches F, ce qui repousse les branches 73 et 83 à l'encontre des ressorts 74 et 84. Cependant, étant donné que les mâchoires 53 et 63 sont fixées, elles ne se déplacent pas, ce qui crée deux espaces libres E entre les parois en regard de ces mâchoires et des fûts en regard. Puis, lorsque le patient cesse ce mouvement d'extension, ou bien que l'effort ainsi exercé est inférieur à la raideur des ressorts 74 et 84, ces derniers repoussent les fûts 12 et 22 dans leur position originelle, illustrée à la figure 2.

La figure 4 illustre une variante de réalisation de l'invention. Sur cette figure 4, les éléments mécaniques qui diffèrent de ceux des figures 1 à 3 y sont représentés par les mêmes numéros, affectés de la référence « prime ».

Dans cette variante de réalisation, la mâchoire 60' n'est plus fixe, comme celle 60 du premier mode, mais au contraire est libre de coulisser le long des tiges 30 et 40. Ce coulissement s'opère à l'encontre de deux ressorts supplémentaires 75 et 85, interposés entre les plots 61' de la mâchoire 60' et des butées 31, 41, montées de manière fixe sur les glissières 30 et 40.

Le montage des mâchoires et des vis, appartenant à l'ensemble conforme à ce second mode de réalisation de l'invention, s'opère de manière analogue à celui décrit en référence au premier mode. Une fois les fûts 12 et 22 introduits dans les oeillets définis par les différentes mâchoires, ces fûts prennent appui contre les mâchoires respectives 50 et 60'.

Etant donné que la mâchoire 60' est désormais mobile, il s'instaure un équilibre entre les forces exercées respectivement par la vis 20, les ressorts « intérieurs » 74 et 84, ainsi que les ressorts « extérieurs » 75 et 85. De façon plus précise, la vis 20 exerce une force tendant à la repousser à l'opposé de la première vis 10, à savoir une contre-réaction par rapport à l'action du chirurgien tendant à les rapprocher. De plus, les ressorts 74 et 84 tendent également à repousser la vis 20, à l'opposé de la vis 10. En revanche, les ressorts 75 et 85 tendent à rapprocher cette vis 20, en direction de la première vis 10.

Dans ce second mode de réalisation, la flexion intervertébrale est désormais possible. Ainsi, lorsque le patient se penche vers l'avant, la branche 53 de la mâchoire fixe maintient immobile la vis 10, alors que la vis 20 repousse la branche 63' de la mâchoire mobile 60' à l'encontre des deux ressorts 75 et 85. Dans le même temps, les ressorts 74 et 84 ont tendance à repousser la branche 83 de la mâchoire 80 en direction du fût 22 de la vis 20, comme cela est illustré sur la figure 5.

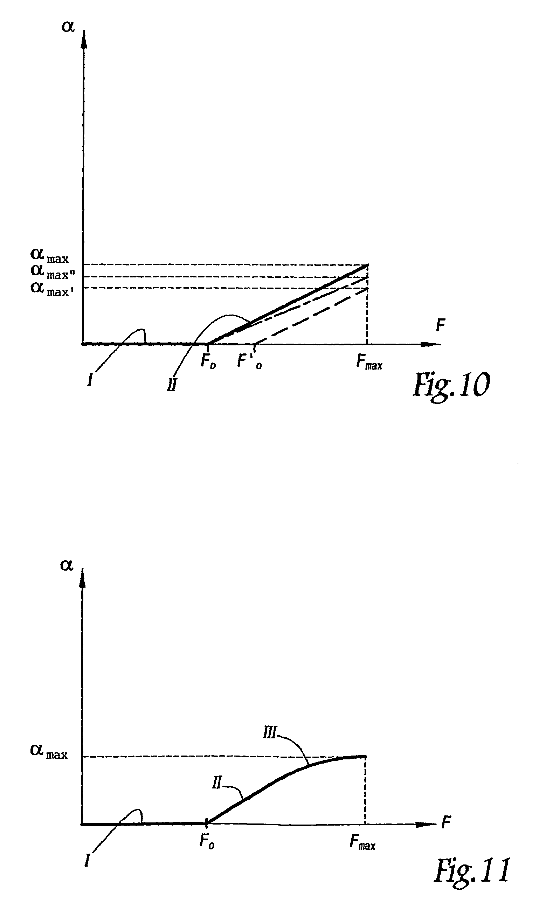

Afin d'expliciter davantage la manière dont s'opère cette flexion intervertébrale, on a représenté à la figure 10 une courbe, illustrant les variations de l'angle α en fonction de la force F. De façon plus précise, F correspond à la force de flexion exercée par le patient, à partir de la position neutre, alors que α correspond à la valeur de la flexion intervertébrale ou, en d'autres termes, à la distance entre les deux vis 10 et 20.

Les ressorts extérieurs 75 et 85 sont précontraints, à savoir qu'on retrouve tout d'abord une zone I dans laquelle le patient exerce une force préalable, afin de vaincre cette précontrainte. En d'autres termes, tant que le patient n'exerce pas une force seuil, notée F₀, il ne parvient pas à « vaincre » cette précontrainte et ainsi n'induit aucune flexion intervertébrale, à savoir que la valeur de l'angle α reste nulle.

Puis, lorsque le patient exerce une force supérieure à cette force seuil F₀, la valeur de l'angle de flexion augmente linéairement avec la force exercée par le patient, selon la caractéristique de raideur du ressort (zone II). Cet angle α augmente alors jusqu'à une valeur notée αₘₐₓ, qui correspond à une valeur Fₘₐₓ, à savoir la force physiologique maximale que peut appliquer le patient. Dans le cadre de l'arthrodèse, dans lequel se situe la présente invention, cette valeur de α est faible comme on l'a vu ci-dessus.

Ce type de courbe permet d'expliquer la distinction, existant entre les notions de raideur et de précontrainte. Ainsi, s'il n'y avait pas de précontrainte, la courbe force-déplacement correspondrait à un tronçon de droite, s'étendant à partir de l'origine. En d'autres termes, la moindre force exercée par le patient tendrait à repousser la vis, à l'encontre des ressorts 75 et 85. Ceci serait peu avantageux, dans la mesure où cette situation conduirait à une instabilité globale du système.

En revanche, plus la précontrainte est élevée, plus la valeur de la force seuil F₀ est importante, avant que le mouvement effectif du patient ne se produise. Sur la courbe, on retrouve en traits pointillés un mode de réalisation dans lequel les ressorts 75 et 85 présentent la même raideur, mais possèdent une précontrainte plus importante. On retrouve une force seuil F_{0'} plus élevée, ainsi qu'un angle maximal αₘₐₓ' plus faible.

En outre, si ces ressorts 75 et 85 présentent des raideurs différentes, ceci influe sur la pente de la droite s'étendant à partir du point F₀. Ainsi, pour une même précontrainte, on retrouve en traits mixtes un agencement dans lequel les ressorts 75 et 85 sont plus raides. En d'autres termes, l'angle maximal αₘₐₓ " que le patient peut atteindre, en exerçant la force physiologique maximale, est moins important que celui αₘₐₓ.

On notera qu'il est possible de régler la valeur de la précontrainte, en modifiant légèrement le mode de réalisation de la figure 4. Ainsi, si on remplace les butées 31 et 41 par des écrous, montés sur une extrémité filetée des tiges 30 et 40, il est alors possible de modifier la position axiale de ces écrous, en les vissant ou en les dévissant. Cette action s'accompagne alors d'une variation correspondante de la précontrainte, impartie aux ressorts 75 et 85.

Lors de l'extension intervertébrale, la coopération des branches 53 et 73, ainsi que de la vis 10, s'opère de manière analogue à ce qui est illustré à la figure 3. En revanche, la vis 20 repousse la branche mobile 83, à l'encontre des ressorts 74 et 84, alors que les ressorts supplémentaires 75 et 85 ont tendance à maintenir l'autre branche mobile 63' au contact du fût 22. En d'autres termes, il n'existe plus d'espace libre entre la branche 63' et le fût 22 sur la figure 6, contrairement à ce qui est représenté à la figure 3.

La figure 7 illustre une variante supplémentaire de réalisation de l'invention. Sur cette figure, les éléments mécaniques qui diffèrent de ceux de la figure 4 y portent les mêmes numéros, affectés de la référence « prime ».

Ce troisième mode de réalisation diffère de celui décrit précédemment, en ce que la mâchoire 50' est désormais mobile, et non plus fixe comme celle 50 des figures 1 et 4. Ainsi, cette mâchoire 50' est montée coulissante sur les glissières 30 et 40, à l'encontre de ressorts 76 et 86, interposées entre les plots 51' de cette mâchoire et des butées d'extrémité 32 et 42.

Le montage des vis 10 et 20 de ce troisième mode de réalisation, dans les oeillets définis par les différentes mâchoires, s'opère de manière analogue à ce qui a été décrit ci-dessus en référence au deuxième mode de réalisation. Une fois cette introduction réalisée, deux équilibres de force s'instaurent, à savoir tout d'abord entre la vis 10, les ressorts 74 et 84, ainsi que ceux 76 et 86 et, d'autre part, entre la vis 20, les ressorts 74 et 84, ainsi que ceux 75 et 85. Ces équilibres de force sont analogues à celui décrit en référence au second mode de réalisation, entre la vis 20, les ressorts intérieurs et les ressorts extérieurs.

Lors de la flexion intervertébrale, le fonctionnement est symétrique, à savoir que la coopération des deux vis avec les quatre mâchoires s'opère de manière analogue, à celle décrite en référence à droite de la figure 5 pour la vis 20 et les deux mâchoires mobiles 60' et 80. Ceci est illustré sur la figure 8, où on retrouve notamment la branche 53' de la mâchoire mobile 50.

De plus, lors de l'extension, le fonctionnement est également symétrique, à savoir que la coopération des deux vis et des quatre mâchoires s'opère de manière analogue à ce qui est décrit à droite de la figure 6, en référence à la vis 20 et aux deux mâchoires mobiles 60' et 80. Une telle extension intervertébrale est illustrée sur la figure 9.

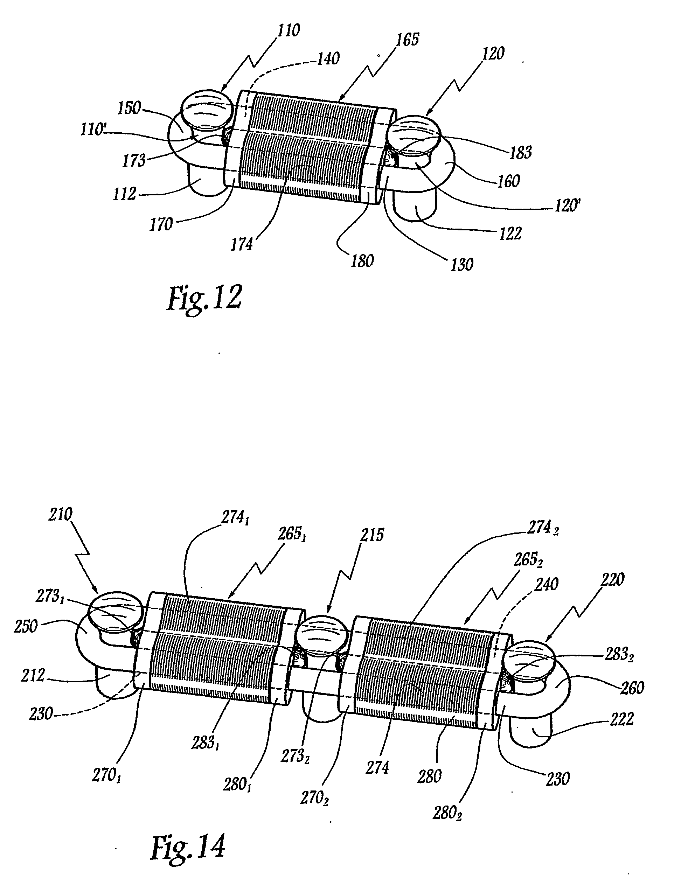

La figure 12 illustre une variante supplémentaire de réalisation de l'invention. Sur cette figure 12 les éléments mécaniques analogues à ceux de la figure 1 y sont affectés des mêmes numéros de référence, augmentés de 100.

Ce mode de réalisation de la figure 10 diffère de celui de la figure 1, en ce que les deux mâchoires mobiles 70 et 80, ainsi que les deux ressorts 74 et 84, sont remplacés par un organe mécanique unique, à savoir un boudin 165. Ce dernier comprend un corps souple 174, réalisé par exemple en un matériau élastomère, ainsi que deux flancs rigides 170 et 180. Chaque flanc est en outre pourvu d'un téton 173, 183, de forme globalement sphérique, propre à coopérer avec les parois en regard d'un fût correspondant 112 ou 122.

De plus, dans le mode de réalisation de la figure 12, les mâchoires fixes 150 et 160 sont formées d'un seul tenant avec le corps des glissières 130 et 140. En d'autres termes, ces dernières sont recourbées, de manière à former deux extrémités reliant mutuellement les glissières, de façon à constituer ces mâchoires 150 et 160. De façon avantageuse, les deux glissières et les deux mâchoires sont ainsi formées par un unique élément filiforme, replié sur lui-même à la façon d'une boucle.

De plus, de façon avantageuse, la distance séparant les glissières 130 et 140, selon un axe perpendiculaire à leur axe principal, est voisine de la section transversale de la vis. Ceci permet aux glissières d'assurer une fonction de guidage de ces vis, afin de les maintenir en bonne position en vue d'une coopération permanente avec les tétons et les extrémités recourbées de la glissière. Dans les modes de réalisation précédents, les vis présentent une section cylindrique constante. Cependant, sur la figure 12, ces vis 110 et 120 présentent un étranglement 110' et 120' de plus faible dimension transversale, coopérant avec les deux glissières en vue du guidage explicité ci-dessus.

Dans le mode de réalisation de la figure 12, les mâchoires fixes empêchent toute flexion intervertébrale. En revanche, lors de l'extension, les vis compriment le corps souple 174, qui joue ainsi le rôle des ressorts 74 et 84. De plus, les flancs 170 et 180 peuvent être assimilés aux mâchoires mobiles 70 et 80, alors que les tétons 173 et 183 peuvent être assimilés aux branches 73 et 83. A cet égard, on notera que la liaison entre chaque téton et le fût correspondant est de type ponctuelle, ce qui autorise une possibilité d'articulation, comme décrit ci-dessus en référence au premier mode de réalisation. Sur la figure 12, les deux mâchoires d'extrémité 150 et 160 sont fixes, comme sur la figure 1. On peut cependant prévoir qu'au moins une mâchoire est mobile, comme sur la figure 4 ou 7, à l'encontre d'au moins un couple de ressorts ou d'au moins un boudin supplémentaire.

Dans les modes de réalisation des figures 4 et 7, faisant intervenir les ressorts « extérieurs » 75, 76, 85 et 86, on peut remplacer ces derniers par un ou deux boudin(s) analogue(s) à celui 165. Dans ces conditions, le corps souple de chaque boudin exerce une fonction analogue à celle d'une paire de ressorts, alors que son flanc rigide présente une fonction similaire à celle des mâchoires mobiles 53' ou 63'.

Dans ce cas, ce corps souple est associé à une valeur de raideur, ainsi que de précontrainte, comme les ressorts. De plus, on peut entourer ce corps souple au moyen d'une chambre rigide, qui constitue ainsi une limite de déformation. Dans ces conditions, la courbe force-déplacement comprend, non plus deux zones comme précédemment, mais trois zones différentes, comme illustré à la figure 11.

On retrouve ainsi tout d'abord des zones I et II, analogues à celles de la figure 10, qui correspondent respectivement à la force préalable exercée par le patient pour vaincre la précontrainte, puis à une variation linéaire du déplacement angulaire en fonction de la force. Enfin, on retrouve une zone III en forme d'asymptote, qui correspond à la venue en butée du corps souple contre les parois de la chambre rigide.

Dans les exemples précédents, on a illustré des ensembles de stabilisation qui relient uniquement deux étages vertébraux. Cependant, on peut prévoir de relier, conformément à l'invention, au moins trois étages vertébraux. A cet effet, deux variantes principales exposées ci-après peuvent être envisagées.

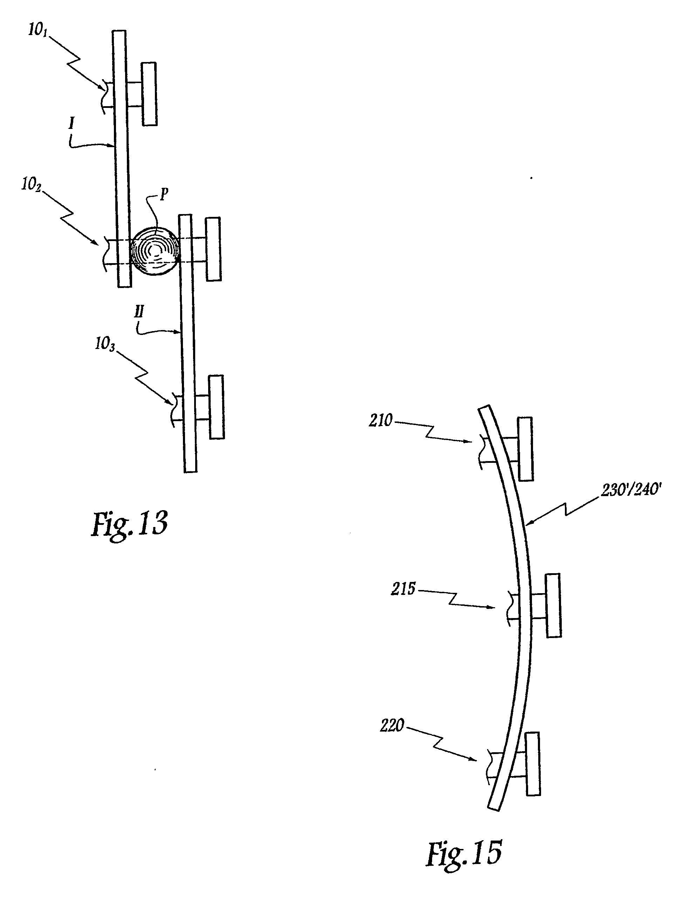

Ainsi, on peut tout d'abord associer plusieurs ensembles extra-discaux, analogues à l'un ou l'autre de ceux décrits ci-dessus, comme cela est représenté sur la figure 13. Sur celle-ci, on retrouve trois vis pédiculaires 10₁, 10₂ et 10₃, ainsi qu'un premier ensemble extra-discal I reliant un premier couple de vertèbres 10₁ et 10₂, et un second ensemble de stabilisation II reliant l'autre couple de vertèbres adjacentes 10₂ et 10₃. Sur cette figure 13, tout comme sur la figure 15 décrite ci-après, chaque ensemble est représenté en vue de côté, de manière très schématique.

De plus, de manière avantageuse, on prévoit d'articuler les deux ensembles I et II l'un par rapport à l'autre. A cet effet, on peut leur associer un organe de séparation sphérique P , de type « perle », qui est en particulier conforme à l'un de ceux décrits et revendiqués dans la demande de brevet français 07 59227, déposée au nom du même Demandeur le 22 novembre 2007, dont le contenu est incorporé par référence. On notera en outre que, sur la figure 13, les glissières des ensembles I et II sont droites par opposition à la forme recourbée qui va être décrite dans l'exemple de réalisation suivant.

A titre d'alternative, on peut envisager de relier au mois trois étages intervertébraux par un unique ensemble de stabilisation, conforme à l'invention, illustré sur la figure 14. Sur cette dernière, les éléments mécaniques analogues à ceux de la figure 12 y sont affectés des mêmes numéros de référence, augmentés de 100.

Le mode de réalisation de la figure 14, pour trois étages vertébraux, diffère de celui de la figure 12, pour deux étages vertébraux, en ce que les glissières 230 et 240 présentent des dimensions axiales plus importantes, de manière à s'étendre au voisinage de ces trois vertèbres. De plus, on retrouve deux mâchoires d'extrémité 250 et 260, s'étendant de part et d'autre du fût 212, 222 des vis pédiculaires d'extrémité 210, 220. Il est en outre prévu deux boudins 265₁ et 265₂, dont chacun relie la vis pédiculaire intermédiaire 215 avec l'une ou l'autre vis pédiculaire d'extrémité 210, 220. Comme dans le mode de réalisation de la figure 12, chaque boudin est pourvu de deux tétons respectivement 273₁, 273₂, 283₁ et 283₂, dont chacun permet l'articulation par rapport à une vis pédiculaire correspondante.

On peut prévoir d'autres variantes de réalisation non représentées, à partir de l'agencement de la figure 14. Ainsi, on peut remplacer au moins un des deux boudins par des ressorts, analogues par exemple à ceux des figures 1, 4 et 7. De plus, sur cette figure 14, les deux mâchoires d'extrémité 250 et 260 sont fixes, comme sur la figure 1. On peut bien évidemment prévoir qu'au moins une de ces mâchoires est mobile, comme dans le mode de réalisation de la figure 4 ou de la figure 7, à l'encontre d'au moins une paire de ressorts supplémentaire, ou encore d'au moins un boudin supplémentaire.

Dans le mode de réalisation de la figure 14, les deux glissières de grande longueur sont droites, comme les glissières plus courtes des premiers modes de réalisation. Cependant, comme l'illustre la figure 15, on peut prévoir de réaliser des glissières 230' et 240' présentant des formes recourbées, en vue de côté. Dans ce cas, elles présentent avantageusement leur concavité dirigée vers la colonne vertébrale. De plus, dans le cas d'un profil recourbé comme celui de la figure 15, il est avantageux de prévoir un jeu fonctionnel entre les parois en regard de la glissière et des deux boudins, afin de faciliter le mouvement de ces derniers le long de ces glissières.

On va maintenant décrire une méthode de pose de l'ensemble de la figure 14. On suppose cependant que les vis 210, 215, 220 ont une section constante, à savoir qu'elles sont dépourvues d'étranglement.

Il s'agit tout d'abord d'implanter les différentes vis pédiculaires 210, 215 et 220, en plaçant sur chacune d'elles une extrémité effilée, par exemple de forme conique. Par ailleurs, on assemble les différents boudins sur les glissières. Du fait de leur nature élastique, et en l'absence des vis, on notera que les différents tétons en regard sont en contact mutuel.

Puis, on rapporte l'ensemble formé par les glissières et les boudins sur les vis, pourvues de leur extrémité pointue. Cette dernière s'insère alors entre les boudins élastiques, puis les repousse de manière à créer une précontrainte. Enfin, lorsque l'ensemble est dans la position de la figure 14, on enlève les différentes extrémités effilées, qu'on remplace avantageusement par un élément analogue à celui P de la figure 13.

Dans ce mode de réalisation, cette perle P permet d'éviter aux mâchoires de glisser le long du fût des vis, à l'opposé des corps vertébraux. De façon avantageuse, on peut prévoir une perle supplémentaire au voisinage des corps vertébraux, afin d'empêcher le glissement en direction de ces derniers. Dans ce cas, ces perles sont introduites le long du fût des vis, avant mise en place des glissières et des boudins.

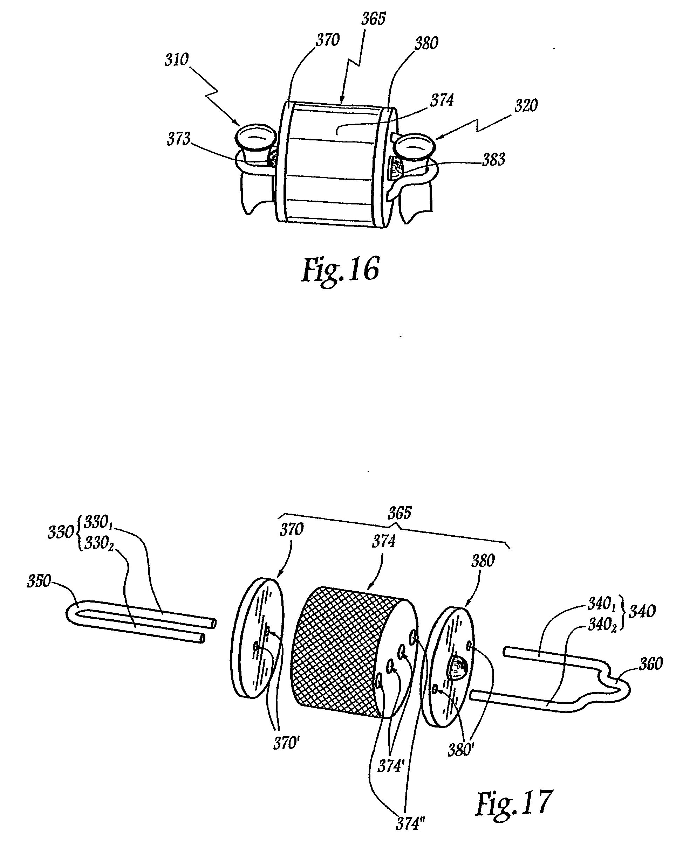

Les figures 16 à 19 illustrent un mode de réalisation supplémentaire de l'invention. Sur ces figures, les éléments mécaniques analogues à ceux de la figure 12 y sont affectés des mêmes numéros de références, augmentés de 200.

On retrouve un boudin 365 comprenant un corps souple cylindrique 374 bordé par deux flancs 370, 380 rigides d'extrémité. Il est en outre prévu quatre glissières, dont les deux premières 330₁, 330₂ appartiennent à un fil métallique recourbé 330, de façon à former une boucle de passage 350 d'une première vis pédiculaire 310. Par ailleurs, les deux autres glissières 340₁, 340₂ appartiennent à un autre fil 340 également recourbé, formant une boucle de passage 360 de l'autre vis pédiculaire 320. Chaque boucle de passage définit une mâchoire au sens de l'invention.

Chaque vis présente une zone médiane de plus faible diamètre, à la manière d'un sablier. Cette zone médiane coopère, de façon articulée, à la fois avec une mâchoire correspondante 350 ou 360, ainsi qu'avec un téton en regard 373 ou 383, comme dans les modes de réalisation précédents.

Le premier fil 330 est monté coulissant dans des orifices 370', ménagés dans le premier flanc 370, adjacent à la boucle 350, puis s'étend également de manière coulissante dans des ouvertures 374' réalisées dans le corps souple. Les extrémités de ce fil sont enfin fixées sur le flanc opposé 380, par tout moyen approprié. A titre de variante, on peut prévoir que ces extrémités traversent ce flanc opposé, de manière coulissante, et sont en outre munies d'un moyen de butée, permettant de retenir ce flanc. En d'autres termes, ce dernier est solidaire en translation de la vis 310 dans le sens d'une compression du corps souple, à savoir d'un rapprochement entre les deux flancs.

De façon analogue, le second fil 340 s'étend de manière coulissante successivement au travers d'orifices 380' ménagés dans le flanc 380 qui est adjacent à la boucle 360, puis d'ouvertures supplémentaires 374" réalisées dans le corps souple. Comme évoqué ci-dessus en référence au premier fil, ce second fil peut être, soit fixé sur le flanc 370 opposé à la boucle 360, soit monté coulissant par rapport à ce dernier, en étant associé à des moyens de butée.

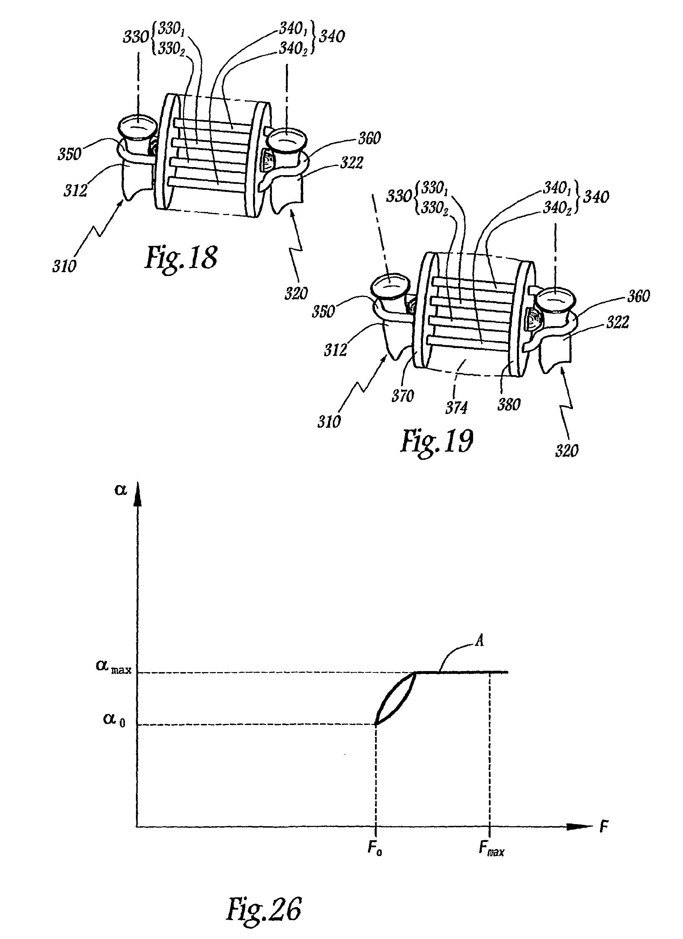

Dans le cas d'une flexion intervertébrale, comme illustré sur la figure 19, ceci induit l'éloignement mutuel des deux vis pédiculaires. Dans ces conditions, la vis droite 320 emmène le flanc gauche 370 vers la droite, alors que la vis gauche 310 emmène le flanc droit 380 vers la gauche, ce qui induit un rapprochement des deux flancs. Dans ces conditions, le corps souple 374 se trouve comprimé, de sorte qu'il joue une fonction d'amortissement et tend à ramener les deux vis l'une vers l'autre, dans leur position originelle.

De plus, lors d'une extension intervertébrale, non représentée sur les figures, chaque boucle a tendance à s'éloigner par rapport à la vis pédiculaire, avec laquelle elle était originellement en contact. Ce déplacement s'accompagne d'une mise en compression correspondante du corps souple, qui assure donc là encore une fonction amortissante et tend à ramener l'ensemble dans sa position initiale de repos.

Comme cela ressort de ce qui précède, non seulement l'extension mais également la flexion intervertébrale conduisent à une compression du corps souple, qui joue par conséquent un rôle d'amortisseur dans l'un ou l'autre des cas. On note ainsi des différences existant entre ce mode de réalisation de la figure 16 et, par exemple, celui de la figure 7. Ainsi, sur cette dernière, chaque couple de mâchoires est associé à deux moyens de rappel distincts, dont chacun travaille dans un sens respectif. En revanche, dans le mode de réalisation de la figure 16, ces deux moyens de rappel sont confondus en un unique moyen de rappel 374, gérant les deux sens opposés de déplacement.

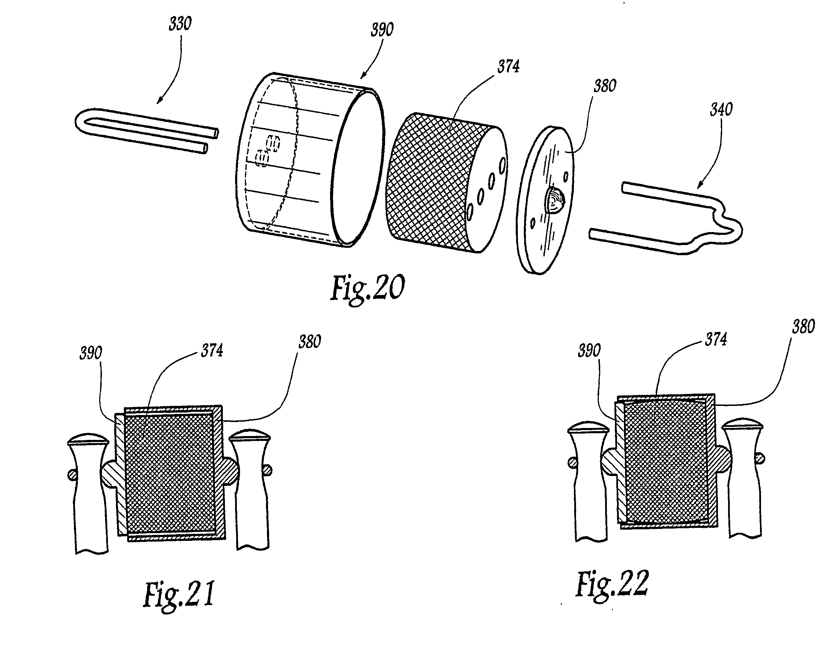

On conçoit que, plus le corps est souple, plus il autorise un mouvement important entre les deux vertèbres. Ainsi, dans le cas de l'arthrodèse, il convient de choisir un corps souple relativement rigide, afin de limiter le débattement intervertébral. On peut également prévoir d'associer à ce corps souple une limite de déformation. Ainsi, on peut par exemple entourer ce corps souple 374 au moyen d'une chambre rigide cylindrique 390, soudée à l'un ou l'autre des flancs, comme illustré à la figure 20.

En référence à la figure 21 dans laquelle le corps souple est en position de repos, les parois de ce dernier s'étendent à distance de celles de la chambre. Dans ces conditions, à un certain stade de la déformation, les parois du corps souple entrent en contact avec celles de la chambre, ce qui interdit tout mouvement supplémentaire (figure 22).

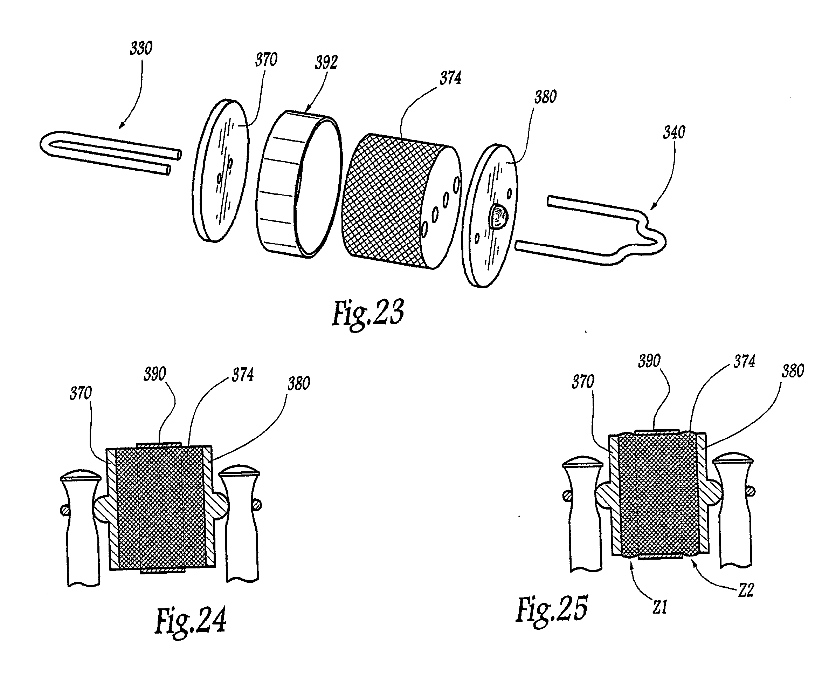

A titre d'alternative, on peut entourer le corps souple 374 par l'intermédiaire d'au moins une bague rigide 392 (figures 23 et 24), s'étendant sur une partie substantielle de ce corps souple. On notera que, par opposition à la création d'une chambre de compression décrite au paragraphe précédent, il n'existe pas d'espace intercalaire entre les parois de la bague et du corps souple, dans la position de repos de ce dernier. En d'autres termes, dans la zone de contact entre cette bague et ce corps souple, ce dernier ne peut se déformer. Dans ces conditions, la zone déformable de ce corps souple correspond uniquement à la partie non entourée par la bague (voir figure 25), soit aux tronçons Z₁ et Z₂.

Dans les deux modes de réalisation précédents, aussi bien la chambre 390 que la bague 392 permettent de limiter la déformation du corps souple 374, ce qui conduit à limiter par conséquent le débattement relatif entre les deux vis pédiculaires. On peut cependant prévoir d'utiliser d'autres moyens permettant de limiter cette course. A titre non limitatif et de manière non représentée, on citera par exemple tout moyen de butée approprié, permettant d'arrêter la course des glissières par rapport aux flancs rigides.

La mise en oeuvre du dispositif des figures 16 et suivantes, pourvue d'une chambre telle que représentée aux figures 20 à 22, est plus particulièrement illustrée à la figure 26, qui est analogue à la figure 10. On retrouve ainsi une courbe illustrant les variations du débattement entre les deux vertèbres en fonction de la force F exercée sur le corps souple 374. L'origine de cette courbe correspond à une position de repos, à savoir exempte de contrainte sur le corps souple, lorsque les vis ne sont pas insérées entre les tétons et une mâchoire correspondante. Puis, lorsqu'on met en place cette vis, ceci conduit à une certaine force de compression sur le corps souple, venant en équilibre avec un certain débattement entre les vis. Ceci correspond au point (F₀ ; α₀) sur la courbe.

Puis, lorsque le patient se penche soit vers l'avant, soit vers l'arrière, comme on l'a vu ci-dessus, ceci conduit dans les deux cas à la compression du corps souple, qui s'oppose à ce mouvement. De plus, la présence de la chambre forme une limite de mouvement, qui est traduite par l'asymptote A correspondant à une inclinaison intervertébrale αₘₐₓ. La courbe de la force en fonction du déplacement est donc limitée entre, d'une part, l'origine « relative » formée par les points F₀ et α₀ et, d'autre part, cette asymptote A.

Dans le mode de réalisation des figures 16 et suivantes, l'organe de rappel est un corps souple massif. Cependant, à titre de variante, on peut prévoir d'utiliser un ressort hélicoïdal, s'étendant selon l'axe reliant les deux vis. Les deux extrémités libres de ce ressort sont alors fixées, par tout moyen approprié, contre les flancs rigides respectifs. Ce mode de réalisation est avantageux, dans la mesure où il permet de réduire les frottements en service, notamment par comparaison avec les frottements liés au mouvement des tiges dans les orifices du corps souple.

Dans les différents modes de réalisation décrits ci-dessus, l'ensemble extra-discal conforme à l'invention comprend au moins deux vis vertébrales. Dans ces conditions, les différentes vis sont en général implantées d'un même côté de la colonne vertébrale, à distance de l'axe vertical médian de cette dernière, en référence au patient debout. On peut également prévoir d'implanter deux jeux de vis vertébrales, de part et d'autre de cet axe médian, chaque jeu de vis appartenant alors à un ensemble extra-discal correspondant.

Cependant, à titre de variante, on peut prévoir d'utiliser, non pas des vis vertébrales, mais des tiges appartenant chacune à un élément de liaison, reliant par exemple deux vis pédiculaires appartenant au même étage vertébral. Dans ces conditions, l'ensemble extra-discal conforme à l'invention comprend au moins deux de ces tiges, placées les unes au dessous des autres sensiblement le long de l'axe vertébral médian, ainsi que des mâchoires, des glissières et des moyens de rappel, comme dans les modes de réalisation précédents.

## Revendications

1. Ensemble extra-discal de stabilisation intervertébrale pour arthrodèse, comprenant
- au moins deux tiges (12, 22; 112, 122; 212, 217, 222; 312, 322), dites tiges vertébrales, propres à coopérer avec au moins deux vertèbres différentes ;
- au moins deux couples de mâchoires (50, 60, 70, 80; 150, 160, 170, 180; 250, 260, 2701, 2702, 2801, 2802; 350, 360, 370, 380), chaque couple de mâchoires étant placé au voisinage d'une tige correspondante ;
- au moins deux glissières (30, 40; 130, 140; 230, 240; 3301, 3302, 3401, 3402) définissant un axe longitudinal dudit ensemble,
- des moyens de rappel (74, 84; 75, 85; 76, 86; 174; 2741, 2742; 374) d'au moins une mâchoire parmi chaque couple de mâchoires, en direction d'une tige vertébrale correspondante,
chaque tige vertébrale étant propre à déplacer, en service, au moins une mâchoire le long des glissières, à l'encontre des moyens de rappel, chaque tige vertébrale étant en outre articulée par rapport à chaque mâchoire,
l'ensemble étant **caractérisé en ce que** les deux glissières (30, 40; 130, 140; 230, 240; 3301, 3302, 3401, 3402) ne sont pas alignées et **en ce qu'**au moins une mâchoire parmi chaque couple de mâchoires est propre à coulisser le long d'au moins deux glissières, selon leur axe longitudinal.

2. Ensemble selon la revendication 1, **caractérisé en ce que** ladite articulation entre la tige et chaque mâchoire s'exerce selon un unique point de contact entre cette tige et les parois en regard de chaque mâchoire.

3. Ensemble selon la revendication 2, **caractérisé en ce qu'**au moins une mâchoire (50, 60, 70, 80) comprend une branche de liaison recourbée (53, 63, 73, 83) selon un rayon de courbure supérieur au rayon de courbure de la tige.

4. Ensemble selon la revendication 3, **caractérisé en ce que** la branche recourbée est solidaire de deux plots (51, 61, 71, 81), montés sur deux glissières (30, 40), éventuellement de manière coulissante.

5. Ensemble selon la revendication 3 ou 4, **caractérisé en ce que** les moyens de rappel comprennent deux ressorts (74, 84, 75, 85, 76, 86), dont chacun est monté sur une glissière correspondante, ces deux ressorts étant propre à rappeler une branche recourbée contre la tige vertébrale.

6. Ensemble selon la revendication 2, **caractérisé en ce qu'**au moins une mâchoire (170, 180) comprend un téton (173, 183) d'articulation contre une tige vertébrale (112, 122) correspondante.

7. Ensemble selon la revendication 6, **caractérisé en ce que** les moyens de rappel comprennent un corps souple massif (174) et le téton (173, 183) appartient à un flanc rigide (170, 180), solidaire de ce corps souple.

8. Ensemble selon l'une quelconque des revendications précédentes, **caractérisé en ce qu'**au moins un couple de mâchoires comprend une première mâchoire montée coulissante le long des glissières, à l'encontre des moyens de rappel, ainsi qu'une seconde mâchoire montée fixe sur les glissières.

9. Ensemble selon l'une quelconque des revendications précédentes, **caractérisé en ce qu'**au moins un couple de mâchoires comprend une première et une seconde mâchoires montées coulissantes le long des glissières, alors que les moyens de rappel comprennent un premier et un second moyen de rappel, chaque mâchoire mobile étant propre à coulisser è l'encontre d'un moyen de rappel correspondant.

10. Ensemble selon la revendication précédente, **caractérisé en ce que** le premier et le second moyens de rappel sont confondus en un moyen de rappel unique, comprenant un organe de rappel central (374), bordé par deux flancs (370, 380) dont chacun définit une première mâchoire respective, **en ce qu'**il est prévu deux paires de glissières (330, 340), chaque paire définissant une seconde mâchoire respective (350, 360), et **en ce que** chaque paire de glissières est propre à coulisser par rapport au flanc adjacent à la mâchoire définie par ces glissières, cette paire de glissières étant en revanche solidaire en translation du flanc opposé, au moins dans le sens d'un rapprochement des deux flancs l'un vers l'autre.

11. Ensemble selon la revendication précédente, **caractérisé en ce que** l'organe de rappel central est un ressort hélicoïdal.

12. Ensemble selon l'une quelconque des revendications précédentes, **caractérisé en ce que** cet ensemble comprend des moyens (390; 392) de limitation de la course entre deux tiges adjacentes, notamment des moyens de limitation de la compression d'un corps déformable (374), appartenant aux moyens de rappel.

13. Ensemble selon la revendication précédente, **caractérisé en ce que** les moyens de limitation de la compression comprennent une chambre rigide (390), s'étendant à distance des parois du corps déformable (374), en position de repos de ce dernier.

14. Ensemble selon la revendication 12, **caractérisé en ce que** les moyens de limitation de la compression comprennent au moins une bague (392) entourant une partie du corps déformable (374), de manière à limiter sa zone de déformation (Z1, Z2).

15. Ensemble selon l'une quelconque des revendications précédentes, **caractérisé en ce qu'**il comprend des moyens permettant d'éviter le glissement des glissières et des mâchoires le long des tiges, le long de l'axe principal de ces dernières, dans au moins un sens.

## Claims

1. Extra-discal assembly for intervertebral stabilisation for arthrodesis, comprising:
- at least two rods (12, 22; 112, 122; 212, 217, 222; 312, 322) called vertebral rods, suitable for working with at least two different vertebrae;
- at least two pairs of jaws (50, 60, 70, 80; 150, 160, 170, 180; 250, 260, 2701, 2702, 2801, 2802; 350, 360, 370, 380), each pair of jaws being placed adjacent to a corresponding rod;
- at least two guideways (30, 40; 130, 140; 230, 240; 3301, 3302, 3401, 3402) defining a longitudinal axis of said assembly,
- retracting means (74, 84; 75, 85; 76, 86; 174; 2741, 2742; 374) for at least one jaw of each pair of jaws, in the direction of a corresponding vertebral rod,
each vertebral rod being suitable, in operation, for shifting at least one jaw along the guideways, against the retraction means, each vertebral rod also being articulated relative to each jaw,
the assembly being **characterized in that** the two guideways (30, 40; 130, 140; 230, 240; 3301, 3302, 3401, 3402) are not aligned, and **in that** at least one jaw of each pair of jaws is suitable for sliding along at least two guideways, according to their longitudinal axis.

2. Assembly according to Claim 1, **characterized in that** said articulation between the rod and each jaw is exerted according to a single point of contact between this rod and the walls facing each jaw.

3. Assembly according to Claim 2, **characterized in that** at least one jaw (50, 60, 70, 80) comprises a linking leg (53, 63, 73, 83) which is curved according to a radius of curvature greater than the radius of curvature of the rod.

4. Assembly according to Claim 3, **characterized in that** the curved leg is integral with two studs (51, 61, 71, 81), which are mounted on two guideways (30, 40), in a sliding manner if required.

5. Assembly according to Claim 3 or 4, **characterized in that** the retraction means comprise two springs (74, 84, 75, 85, 76, 86), each of which is mounted on a corresponding guideway, these two springs being suitable for retracting a curved leg against the vertebral rod.

6. Assembly according to Claim 2, **characterized in that** at least one jaw (170, 180) includes a lug (173, 183) for articulation against a corresponding vertebral rod (112, 122).

7. Assembly according to Claim 6, **characterized in that** the retraction means comprise a solid flexible body (174), and the lug (173, 183) belongs to a rigid flank (170, 180), which is integral with this flexible body.

8. Assembly according to any one of the preceding claims, **characterized in that** at least one pair of jaws comprises a first jaw which is mounted sliding along the guideways, against the retraction means, and a second jaw which is mounted fixed on the guideways.

9. Assembly according to any one of the preceding claims, **characterized in that** at least one pair of jaws comprises a first and a second jaw which are mounted sliding along the guideways, whereas the retraction means comprise a first and a second retraction means, each movable jaw being suitable for sliding against a corresponding retraction means.

10. Assembly according to the preceding claim, **characterized in that** the first and the second retraction means are combined into a single retraction means, comprising a central retraction component (374), edged by two flanks (370, 380), each of which defines a respective first jaw, **in that** two pairs of guideways (330, 340) are provided, each pair defining a second respective jaw (350, 360), and **in that** each pair of guideways is suitable for sliding relative to the flank which is adjacent to the jaw defined by these guideways, this pair of guideways being on the other hand integral in translation with the opposite flank, at least in the sense of bringing the two flanks nearer each other.

11. Assembly according to the preceding claim, **characterized in that** the central retraction component is a helical spring.

12. Assembly according to any one of the preceding claims, **characterized in that** this assembly includes means (390; 392) for limiting the travel between two adjacent rods, in particular means for limiting the compression of a deformable body (374) which belongs to the retraction means.

13. Assembly according to the preceding claim, **characterized in that** the means for limiting the compression include a rigid chamber (390), which extends at a distance from the walls of the deformable body (374) in the resting position of the latter.

14. Assembly according to Claim 12, **characterized in that** the means for limiting the compression include at least one bush (392), which surrounds part of the deformable body (374) so as to limit its deformation zone (Z1, Z2).

15. Assembly according to any one of the preceding claims, **characterized in that** it includes means which make it possible to avoid the sliding of the guideways and jaws along the rods, along the principal axis of the latter, in at least one direction.

## Patentansprüche

1. Extra-diskale Anordnung zur Bandscheibenstabllisierung für Arthrodese, umfassend:
- mindestens zwei als Spinalstangen bezeichnete Stangen (12, 22; 112, 122; 212, 217, 222; 312, 322), die zum Zusammenwirken mit mindestens zwei verschiedenen Wirbeln geeignet sind;
- mindestens zwei Klemmbackenpaare (50, 60, 70, 80; 150, 160, 170, 180; 250, 260, 2701, 2702, 2801, 2802; 350, 360, 370, 380), wobei jedes Klemmbackenpaar nahe einer der zugeordneten Stangen angeordnet ist;
- mindestens zwei Gleitschienen (30, 40; 130, 140; 230, 240; 3301, 3302, 3401, 3402), die eine Längsachse der Anordnung definieren;
- Mittel (74, 84; 75, 85; 76, 86; 174; 2741, 2742; 374) zum Zurückstellen mindestens einer Klemmbacke aus jedem Klemmbackenpaar in Richtung auf eine entsprechende Spinalstange,
wobei jede Spinalstange dazu geeignet ist, um im Einsatz mindestens eine Klemmbacke an den Gleitschienen entlang gegen die Rückstellmittel zu verschieben, wobei jede Spinalstange ferner im Verhältnis zu jeder Klemmbacke angelenkt ist,
wobei die Anordnung **dadurch gekennzeichnet ist, dass** die beiden Gleitschienen (30, 40; 130,140; 230, 240; 3301, 3302, 3401, 3402) nicht ausgerichtet sind, und dass mindestens eine Klemmbacke aus jedem der Klemmbackenpaare geeignet ist, an mindestens zwei Gleitschienen entlang ihrer Längsachse zu gleiten.

2. Anordnung nach Anspruch 1, **dadurch gekennzeichnet, dass** die Anlenkung zwischen der Stange und jeder Klemmbacke an einem einzigen Kontaktpunkt zwischen dieser Stange und den Wänden gegenüber jeder Klemmbacke ausgeübt wird.

3. Anordnung nach Anspruch 2, **dadurch gekennzeichnet, dass** mindestens eine Klemmbacke (50, 60, 70, 80) einen Verbindungsschenkel (53, 63, 73, 83) umfasst, der in einem Krümmungsradius größer als der Krümmungsradius der Stange gekrümmt ist.

4. Anordnung nach Anspruch 3, **dadurch gekennzeichnet, dass** der gekrümmte Schenkel mit zwei Stiften (51, 61, 71, 81) fest verbunden Ist, die auf zwei Gleitschienen (30, 40) gegebenenfalls gleitend montiert sind.

5. Anordnung nach Anspruch 3 oder 4, **dadurch gekennzeichnet, dass** die Rückstellmittel zwei Federn (74, 84, 75, 86, 76, 86) umfassen, von denen jede auf einer entsprechenden Gleitschiene montiert ist, wobei diese beiden Federn dazu geeignet sind, einen gekrümmten Schenkel an die Spinalstange zurückzustellen.

6. Anordnung nach Anspruch 2, **dadurch gekennzeichnet, dass** mindestens eine Klemmbacke (170, 180) einen Ansatz (173, 183) zur Anlenkung an einer entsprechenden Spinalstange (112, 122) umfasst.

7. Anordnung nach Anspruch 6, **dadurch gekennzeichnet, dass** die Rückstellmittel einen massiven biegsamen Körper (174) umfassen und der Ansatz (173, 183) zu einer unbiegsamen Flanke (170, 180) gehört, die mit diesem biegsamen Körper fest verbunden ist.

8. Anordnung nach einem der vorhergehenden Ansprüche, **dadurch gekennzeichnet, dass** mindestens ein Klemmbackenpaar eine erste Klemmbacke, die an Gleitschienen entlang gegen Rückstellmittel gleitend montiert ist, sowie eine zweite Klemmbacke, die auf den Gleitschienen fest montiert ist, umfasst.

9. Anordnung nach einem der vorhergehenden Ansprüche, **dadurch gekennzeichnet, dass** mindestens ein Klemmbackenpaar eine erste und eine zweite Klemmbacke umfasst, die an den Gleitschienen entlang gleitend montiert sind, während die Rückstellmittel ein erstes und ein zweites Rückstellmittel umfassen, wobei jede bewegliche Klemmbacke dazu geeignet ist, um gegen ein entsprechendes Rückstellmittel zu gleiten.

10. Anordnung nach dem vorherigen Anspruch, **dadurch gekennzeichnet, dass** die ersten und die zweiten Rückstellmittel in einem einzigen Rückstellmittel zusammengelegt sind, das ein zentrales Rückstellorgan (374) umfasst, das von zwei Flanken (370, 380) eingefasst ist, von denen jede eine jeweilige erste Klemmbacke definiert, dass zwei Gleitschienenpaare (330, 340) bereitgestellt werden, wobei jedes Paar eine jeweilige zweite Klemmbacke (350, 360) definiert, und dass jedes Gleitschienenpaar dazu geeignet ist, um im Verhältnis zu der Flanke neben der durch diese Gleitschienen definierten Klemmbacke zu gleiten, wobei dieses Gleitschienenpaar hingegen mit der gegenüberliegenden Flanke transllationsmäßig fest verbunden ist, zumindest in Richtung einer Annäherung der beiden Flanken aneinander.

11. Anordnung nach dem vorhergehenden Anspruch, **dadurch gekennzeichnet, dass** das zentrale Rückstellorgan eine Schraubenfeder ist.

12. Anordnung nach einem der vorhergehenden Ansprüche, **dadurch gekennzeichnet, dass** diese Anordnung Mittel (390; 392) zur Begrenzung des Weges zwischen zwei angrenzenden Stangen umfasst, insbesondere Mittel zum Begrenzen der Kompression eines verformbaren Körpers (374), der zu den Rückstellmitteln gehört.

13. Anordnung nach dem vorhergehenden Anspruch, **dadurch gekennzeichnet, dass** die Mittel zum Begrenzen der Kompression eine unbiegsame Kammer (390) umfassen, die sich in einem gewissen Abstand von den Wänden des verformbaren Körpers (374) in der Ruheposition desselben erstrecken.

14. Anordnung nach Anspruch 12, **dadurch gekennzeichnet, dass** die Mittel zum Begrenzen der Kompression mindestens einen Ring (392) umfassen, der einen Tell des verformbaren Körpers (374) umgilbt, um seine Verformungszone (Z1, Z2) zu begrenzen.

15. Anordnung nach einem der vorhergehenden Ansprüche, **dadurch gekennzeichnet, dass** sie Mittel umfasst, die es ermöglichen, das Gleiten der Gleitschienen und der Klemmbacken an den Stangen, an der Hauptachse derselben entlang, in mindestens einer Richtung zu vermeiden.
